# EUROPÄISCHE PATENTSCHRIFT

(11) **EP 1 313 692 B1**
(45) Veröffentlichungstag und Bekanntmachung des Hinweises auf die Patenterteilung: **20.07.2005**
(21) Anmeldenummer: 01965143.9
(22) Anmeldetag: 23.07.2001
(51) Int. Cl.: C07C 49/647, C07C 49/587, C07C 49/385, C07C 45/65, C07C 45/59, C07C 45/67, C07C 45/62, C11B 9/00, A61K 7/46

(54) **NEUE MAKROCYCLISCHE KETONE**
NOVEL MACROCYCLIC KETONES
NOUVELLES CETONES MACROCYCLIQUES

(30) Priorität: 04.08.2000 DE 10038021
(43) Veröffentlichungstag der Anmeldung: 28.05.2003
(73) Patentinhaber: Symrise GmbH & Co. KG, 37603 Holzminden (DE)
(72) Erfinder: EH, Marcus, 37603 Holzminden (DE); WÖHRLE, Ingo, 37603 Holzminden (DE)
(74) Vertreter: Eisenführ, Günther, Dipl.-Ing.
(86) Internationale Anmeldenummer: PCT/EP2001/008471
(87) Internationale Veröffentlichungsnummer: WO 2002/012157

(56) Entgegenhaltungen:
- EP-A- 0 025 869
- DE-C- 532 654
- US-A- 3 718 696
- US-A- 3 923 699
- US-A- 4 885 397

## Beschreibung

Die Erfindung betrifft neue Niederalkyl- und Niederalkyliden-substituierte, gesättigte bzw. ungesättigte Cyclohexadecanone, ihre Herstellung und Verwendung in der funktionellen Parfümerie und in der Feinparfümerie.

Verbindungen mit Moschusgeruch sind begehrte Komponenten in der Duftstoffindustrie. Sie zeichnen sich sowohl durch ihre Eigenschaft, Parfümkompositionen Ausstrahlung zu verleihen als auch durch ihre Fähigkeit als Fixateur zu wirken, aus. Somit kommen heutzutage Moschus Riechstoffe in vielen Parfümkompositionen zum Einsatz.

Die Klasse der naturähnlichen makrocyclischen Moschusriechstoffe wird in Zukunft immer mehr an Bedeutung gewinnen, da die synthetischen Moschusverbindungen der nitroaromatischen und polycyclischen Reihe persistent und lipophil sind, so dass diese Verbindungen sich in aquatischen Nahrungsketten und Fettgewebe anreichern (Ernährungs-Umschau 1996, 43, 442 bis 449; Ernährungs-Umschau 1997, 44, 4 bis 9).

Typische Moschus-Riechstoffe zeichnen sich durch einen makrocyclischen Ring mit 13 bis 17 C-Atomen aus, welcher als funktionelle Gruppe ein Keton oder einen Ester trägt. Bekanntermaßen ist die Stabilität von Ketonen in sauren, wie in alkalischen Medien größer als die der entsprechenden Lactone. Darüber hinaus ist auch eine Alkylsubstitution, hierbei bevorzugt eine Methylsubstitution, im makrocyclischen Ring möglich.

In der europaischen Anmeldung EP-A-0 025 869 wird die Verbindung 3-Methylcyclohexadecen-5-on-1 als Riechstoff offenbart, mit Moschusnote.

Ein in der Parfümerie besonders bevorzugter Riechstoff ist das sogenannte Muscon.

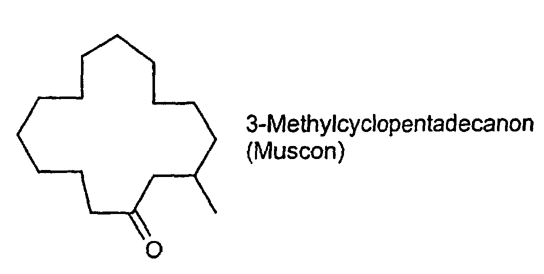

Muscon ist einer der wichtigsten Inhaltsstoffe der Duftdrüse des Moschustieres und seit seiner Strukturaufklärung im Jahr 1926 (Helv. Chim. Acta, 9, 230, 1926) sind zahlreiche Synthesen sowohl zu racemischem, wie auch zu optisch reinem Muscon veröffentlicht worden (Fragrance Chemistry. The Science of the Sense of Smell, ed. E.T. Theimer, Academic Press, 1982, Seiten 444 bis 469). Die Synthesen beinhalten viele Reaktionsstufen und benötigen teure Ausgangsprodukte und/oder Reaktionskomponenten. Somit sind die Einsatzmengen des teuren Muscons in der funktionellen wie auch in der Feinparfümerie beschränkt.

In US-A-4 885 397 werden macrocyclische-Ketone offenbart und deren Eigenschaft als Riechstoffe. In US-A-3 923 699 wird cyclohexadecenone-5 offenbart, und dessen Verwendung als Riechstoff, mit Moschusnote. Dokument US-A-3 718 696 offenbart 1-, und 2-Methylcyclohexadecenone und deren Verwendung als Riechstoff, mit Moschus Eigenschaften.

Es besteht daher ein dringender Bedarf an weiteren makrocyclischen Verbindungen mit Muscon-Note, die in einer effizienten Synthese aus preisgünstigen Ausgangsprodukten herstellbar sind und darüber hinaus mit ihren originellen Dufteigenschaften die Möglichkeiten des Parfümeurs erweitern.

Es bestand daher die Aufgabe, neue preisgünstige makrocyclische Ketone mit Muscon-Note zu finden.

Es wurden neue makrocyclische Ketone der allgemeinen Formel 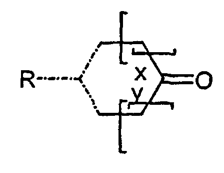 gefunden, worin
- R: eine C₁-C₆-Alkyl- oder C₁-C₆-Alkylidengruppe bedeutet,
x = 5 und y = 7, oder
x = 6 und y = 6 sind, und
die unterbrochenen Linien unabhängig voneinander C-C-Einfach- oder eine C=C-Doppelbindung
bedeuten.

Überraschenderweise wurde gefunden, dass die neuen makrocyclischen Ketone einen starken und typischen Muscon-Geruch aufweisen. Der Befund ist insofern überraschend, als dass die als Riechstoffe bereits bekannten Gyclohexadecanon und 8-Cyclohexadecenon nicht musconartig riechen.

Bevorzugt sind makrocyclische Ketone der Formel 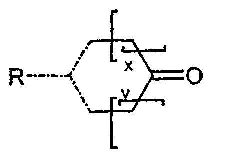 worin
- R: Methyl oder Ethyl bedeutet und
x und y und die unterbrochenen Linien die oben genannte Bedeutung haben.

Als C₁-C₆-Alkyl sind Beispielsweise: Methyl, Ethyl, Propyl, iso-Propyl, Butyl, iso-Butyl, Pentyl, iso-Pentyl, Hexyl und iso-Hexyl genannt.

Bevorzugte Reste sind Methyl und Ethyl.

Besonders bevorzugter Rest ist Methyl.

Als C₁-C₆-Alkyliden sind beispielsweise Methyliden, Ethyliden, Propyliden, isoPropyliden, Butyliden, iso-Butyliden, Pentyliden, iso-Pentyliden, Hexyliden und iso-Hexyliden genannt.

Bevorzugte Reste sind Methyliden und Ethyliden.

Besonders bevorzugter Rest ist Methyliden.

Im einzelnen seien die folgenden Cyclohexadecenone oder Cyclohexadecanone genannt:
8-Methylencyclohexadecanon
9-Methylencyclohexadecanon
8-Ethylencyclohexadecanon
9-Ethylencyclohexadecanon
8-Methyl-(E/Z)-7/-(E/Z)-8-cyclohexadecenon
9-Methyl-(E/Z)-8-cyclohexadecenon
8-Ethyl-(E/Z)-7/-(E/Z)-8-cyclohexadecenon
9-Ethyl-(E/Z)-8-cyclohexadecenon
8-Methylcyclohexadecanon
9-Methylcyclohexadecanon
8-Ethylcyclohexadecanon
9-Ethylcyclohexadecanon

Es wurde ein Verfahren zur Herstellung der erfindungsgemäßen C₁-C₆-Alkyl- oder C₁-C₆-Alkyliden- substituierten Cyclohexadecenone oder Cyclohexadecanone der Formel 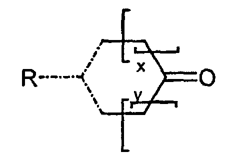 gefunden,
worin
die unterbrochenen Linien unabhängig voneinander C-C-Einfach- oder eine C=C-Doppelbindung bedeuten
- R: eine C₁-C₆-Alkyl- oder C₁-C₆-Alkylidengruppe bedeutet,
x = 5 und y = 7, oder
x = 6 und y = 6 sind,
das dadurch gekennzeichnet ist, dass als Edukt jeweils Cyclohexadecandion der Formel 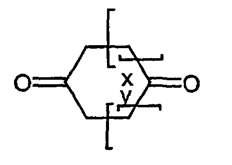 worin
x und y die oben genannte Bedeutung haben,
eingesetzt wird,
und in einem ersten Schritt mit einem C₁-C₆-Alkyl-Triphenylphosphonium-halogenid und einer starken Base in einem aprotischen Lösungsmittel umgesetzt wird und die so erhaltenen C₁-C₆-Alkylidencyclohexadecanone gegebenenfalls isomerisiert und hydriert werden.

Cyclohexadecandion (J. Org. Chem., 1968, 33, 4541; US 3,935,270) wird hierbei im ersten Verfahren zunächst mit 0,1 bis 2,0 Äquivalenten, bevorzugt mit 0,2 bis 1,6 Äquivalenten und besonders bevorzugt mit 0,3 bis 1,2 Äquivalenten C₁-C₆-Alkyltriphcnylphosphoniumhalogenid und 0,1 bis 2,0 Äquivalenten, bevorzugt mit 0,2 bis 1,6 Äquivalenten und besonders bevorzugt mit 0,3 bis 1,2 Äquivalenten einer starken Base (z.B. KO^{t}Bu, n-BuLi etc.) in einem aprotischen Lösungsmittel (z.B. Diethylether, THF, Toluol, Hexan etc.) unter Rückfluss umgesetzt (Chem. Rev., 1989, 89, 863; Synth. Commun., 1985, 15, 855). Das erhaltene Produktgemisch aus Cyclohexadecandion, C₁-C₆-Alkylidencylohexadecanon und Di-C₁-C₆-Alkylidencyclohexadecan wird aufgereinigt (z.B. durch Destillation oder Chromatographie), so dass man die erfindungsgemäßen C₁-C₆-Alkylidencyclohexadecanone in reiner Form erhält.

Die auf oben beschriebene Weise synthetisierten neuartigen C₁-C₆-Alkylidencyclohexadecanone können zu zwei weiteren neuen Verbindungsklassen derivatisiert werden. Einerseits werden die erfindungsgemäßen C₁-C₆-Alkylidencyclohexadecanone in Toluol unter Zugabe von 0,01 bis 2,0 Äquivalenten, bevorzugt 0,05 bis 1,0 Äquivalenten und besonders bevorzugt 0,1 bis 0,5 Äquivalenten p-Toluolsulfonsäure auf eine Temperatur von 80°C bis 110°C und bevorzugt 100°C bis 110°C (Tetrahedron, 1998, 54, 865) erhitzt und isomerisieren unter diesen Bedingungen zu den erfindungsgemäßen C₁-C₆-Alkylcyclohexadecenonen. Andererseits werden die erfindungsgemäßen C₁-C₆-Alkylidencyclohexadecanone unter Normal-Wasserstoffdruck bei einer Temperatur von 25°C bis 70°C und bevorzugt 40°C bis 60°C in Essigsäureethylester und Pd/C als Hydrierkatalysator zu den erfindungsgemäßen C₁-C₆-Alkylcyclohexadecanonen hydriert.

Das erfindungsgemäße erste Verfahren kann am Beispiel des 9-Methylencyclohexadecanons und Folgeprodukten durch das folgende Formelschema erläutert werden:

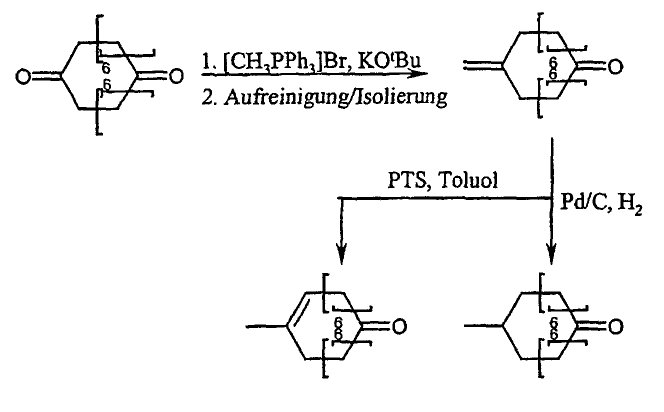

Ein Alternativverfahren zur Herstellung der erfindungsgemäßen C₁-C₆-Alkyl- oder C₁-C₆-Alkyliden substituierten Cyclohexadecanone der Formel 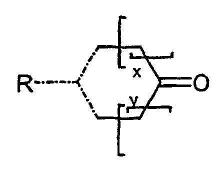 worin
die unterbrochenen Linien unabhängig voneinander C-C-Einfach- oder eine C=C-Doppelbindung bedeuten,
- R: eine C₁-C₆-Alkyl- oder C₁-C₆-Alkylidengruppe bedeuten,
x = 5 und y = 7, oder
x = 6 und y = 6 sind,
ist dadurch gekennzeichnet, dass in einem ersten Schritt die Ketofunktion über ein Ethylenacetal geschützt wird. Nachfolgende Wittig-Reaktion und Entschützung liefert die C₁-C₆-Alkylidencyclohexadecanone, welche gegebenenfalls isomerisiert und hydriert werden.

In dem Alternativverfahren wird Cyclohexadecandion (J. Org. Chem., 1968, 33, 4541; US 3,935,270) mit 0,1 bis 2,0 Äquivalenten, bevorzugt mit 0,3 bis 1,5 Äquivalenten und besonders bevorzugt mit 0,8 bis 1,2 Äquivalenten Ethylenglykol und 0,01 Äquivalenten bis 0,5 Äquivalenten, bevorzugt 0,05 bis 0,2 Äquivalenten p-Toluolsulfonsäure in Toluol am Wasserabscheider umgesetzt. Das erhaltene Verbindungsgemisch aus Cyclohexadecandion, Dioxaspiroeicosanon und Tetraoxadispirotetracosan wird einerseits als Verbindungsgemisch oder andererseits nach Aufreinigung, z.B. Destillation oder Chromatographie, als reines Dioxaspiroeicosanon in die nachfolgende Wittig-Reaktion eingesetzt.

Das nach dem 1. Schritt erhaltene Verbindungsgemisch aus Cyclohexadecandion, Dioxaspiroeicosanon und Tetraoxadispirotetracosan wird mit 0,1 bis 2,0 Äquivalenten, bevorzugt mit 0,3 bis 1,5 Äquivalenten und besonders bevorzugt mit 0,8 bis 1,2 Äquivalenten (pro Mol Ketofunktion) C₁-C₆-Alkyltriphenylphosphoniumhalogenid und 0,1 bis 2,0 Äquivalenten, bevorzugt 0,3 bis 1,5 Äquivalenten und besonders bevorzugt 0,8 bis 1,2 Äquivalenten (pro Mol Ketofunktion) einer starken Base (z.B. KO^{t}Bu, n-BuLi etc.) in einem aprotischen Lösungsmittel (z.B. Diethylether, THF, Toluol, Hexan etc.) unter Rückfluss umgesetzt. Das erhaltene Produktgemisch, bestehend aus Di-C₁-C₆-Alkylidencyclohexadecan, C₁-C₆-Alkylidendioxaspiroeicosan und Tetraoxadispirotetracosan wird aufgereinigt (z.B. durch Chromatographie oder Destillation), so dass ausschließlich C₁-C₆-Alkylidendioxaspiroeicosan in die nachfolgende Acetalspaltung eingesetzt wird.

Das C₁-C₆-Alkylidendioxaspiroeicosan wird in einem 3:1 Aceton/Wasser Gemisch unter Zugabe von 0,01 bis 0,8 Äquivalenten und bevorzugt 0,1 bis 0,5 Äquivalenten Pyridinium-p-toluolsulfonat umgesetzt, so dass die erfindungsgemäßen C₁-C₆-Alkylidencyclohexadecanone resultieren.

Die nachfolgenden Derivatisierungen (Doppelbindungsisomerisierung und Doppelbindungshydrierung) werden analog den Beschreibungen des ersten Verfahrens durchgeführt.

Für den Fall, dass man nach der ersten Stufe reines Dioxaspiroeicosanon zur Verfügung hat, so wird dieses mit 1,0 bis 2,0 Äquivalenten und bevorzugt mit 0,8 bis 1,2 Äquivalenten C₁-C₆-Alkyltriphenylphosphoniumhalogenid und 0,8 bis 1,2 Aquivalenten und bevorzugt 1,0 bis 1,4 Äquivalenten einer starken Base (z.B. KO^{t}Bu, n-BuLi etc.) in einem aprotischen Lösungsmittel (z.B. Diethylether, THF, Toluol, Hexan etc.) umgesetzt.

Die nachfolgende Acetalspaltung, Doppelbindungsisomerisierung oder Doppelbindungshydrierung findet nach den oben beschriebenen Prozeduren statt.

Das erfindungsgemäße Verfahren kann am Beispiel des 9-Methylencyclohexadecanons und Folgeprodukten durch das folgende Formelschema erläutert werden:

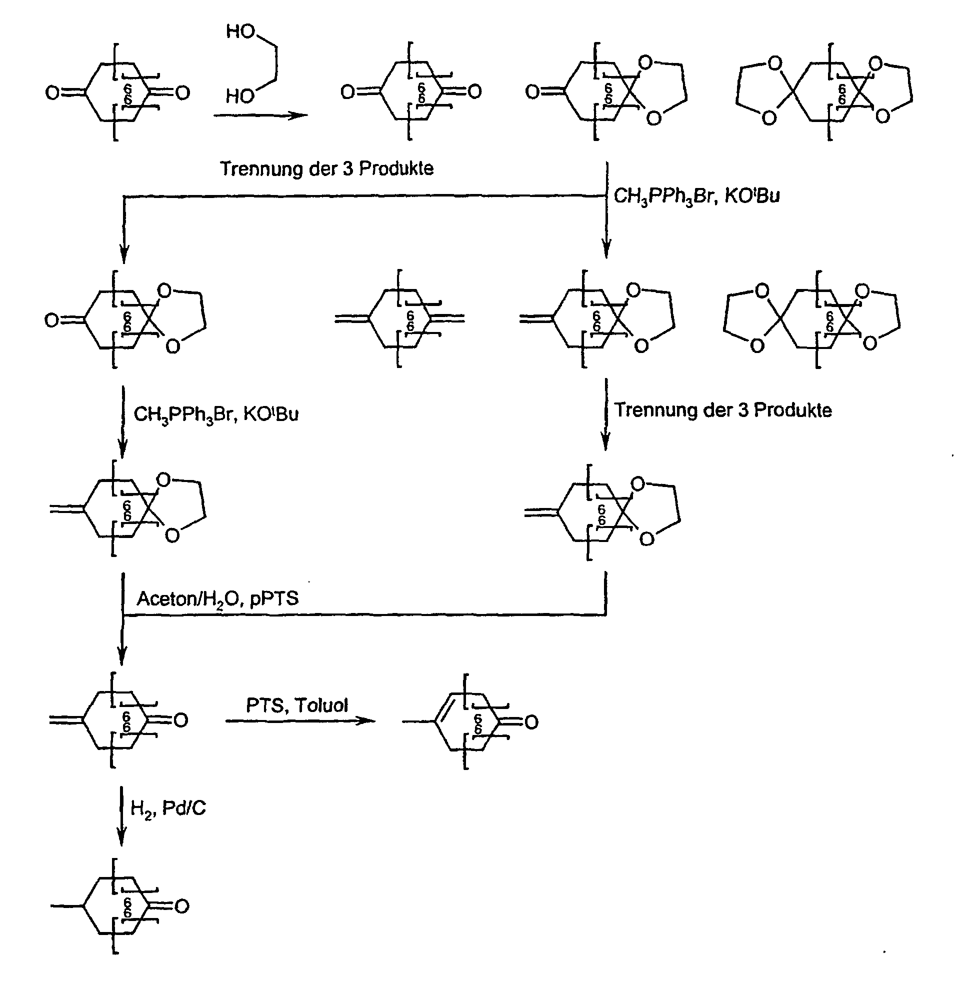

Die erfindungsgemäßen makrocyclischen Ketone können dabei als Einzelstoffe in einer Vielzahl von Produkten verwendet werden; besonders vorteilhaft lassen sie sich mit anderen Riechstoffen zu neuartigen Parfümkompositionen kombinieren.

Durch die Verwendung der erfindungsgemäßen makrocyclischen Ketone lassen sich in der Regel bereits in geringer Dosierung in den resultierenden Parfümkompositionen feine, erogene Moschusnoten erzielen, wobei der geruchliche Gesamteindruck auffallend harmonisiert, die Ausstrahlung wahrnehmbar erhöht und die Fixierung, d.h. das Haftvermögen des Parfümöles, deutlich verstärkt wird.

Beispiele für Riechstoffe, mit denen die erfindungsgemäßen makrocyclischen Ketone vorteilhaft kombiniert werden können, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 3^{rd}. Ed., Wiley-VCH, Weinheim 1997.

Im einzelnen seien genannt:

Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citiiodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon bzw. daraus isolierten Inhaltsstoffen;

Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinoat; Methyl-2-noninoat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavandulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpineol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedemholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha-3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether, (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cylopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cylohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl}-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5 bzw. 6-indenylacetat; 4,7-Methano-3a,4,5,6,7,7a-hexahydro-5 bzw. 6-indenylpropionat; 4,7-Methano-3a,4,5,6,7,7a-hexahydro-5 bzw. 6-indenylisobutyrat; 4,7-Methanooctahydro-5 bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether, 2-Phenylethyl-1-ethoxyethylether; Phenylacetaldehyddimethylacetal; Phenylacetaldehyddiethylacetal; Hydratropaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methyl-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Iso-butyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid, 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die die erfindungsgemäßen makrocyclischen Ketone enthaltenden Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können die die erfindungsgemäßen makrocyclischen Ketone enthaltenden Parfümöle an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die die erfindungsgemäßen makrocyclischen Ketone enthaltenden Parfümöle können auch mikroverkapselt, sprühgetrocknet, als Einschluß-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus Polyurethan-artigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, gegebenenfalls in einem geeigneten Lösungsmittel, z.B. Isopropanol, erhalten werden.

In Parfümkompositionen beträgt die eingesetzte Menge der erfindungsgemäßen makrocyclischen Ketone 0,05 bis 50 Gew.-%, vorzugsweise 0,5 bis 20 %, bezogen auf das gesamte Parfümöl.

Die die erfindungsgemäßen makrocyclischen Ketone enthaltenden Parfümöle können in konzentrierter Form, in Lösungen oder in oben beschriebener modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigem, Geschirrspülmittel, Bad- und Sanitärreinigern, Scheuermilch, festen und flüssigen WC-Reinigem, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukten wie z.B. Haarsprays, Haargelen, festigende Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Larnpenölen, Räucherstäbchen, Insektiziden, Repellentien, Treibstoffen.

Ein Schwerpunkt der Verwendung der erfindungsgemäßen makrocyclischen Ketone liegt wegen ihrer Stabilität im alkalischen Bereich bei der Parfümierung von Seifen und Waschmitteln. Bei der Verwendung in Waschmittelparfümierungen zeichnen sich die erfindungsgemäßen makrocyclischen Ketone durch eine im Vergleich zu bisher verwendeten Riechstoffen erhöhte Substantivität, d.h. durch ein verstärktes Aufziehvermögen und eine erhöhte Haftung des Riechstoffs auf der gewaschenen Faser, aus.

Die folgenden Beispiele erläutern die Erfindung:

### Beispiele

### Beispiel 1:

### 1,4-Dioxaspiro-[4.15]-eicosan-13-on

Zu einer Lösung aus 44,0 g (175 mmol) 1,9-Cyclohexadecandion in 125 ml Toluol fügt man 13,1 g (210 mmol) Ethylenglykol und 500 mg (2,5 mmol) p-Toluolsulfonsäure hinzu. Jetzt erhitzt man am Wasserabscheider bis sich keine sichtbaren Mengen Wasser mehr abscheiden. Anschließend wäscht man mit ges. NaHCO₃-Lösung, trennt die Phasen, trocknet die organische Phase über Na₂SO₄, filtriert ab und entfernt das Lösungsmittel am Rotationsverdampfer. Man erhält 57,7 g eines Feststoffes aus 3 Komponenten mit folgendem GC-Gehalt: 1,9-Cyclohexadecandion (33,7 %); 1,4-Dioxaspiro-[4.15]-eicosan-13-on (52,5 %); 1,4,14,17-Tetraoxadispiro-[4.7.4.7]-tetracosan (13,2 %).

Im folgenden wird sowohl das 3-Komponentengemisch wie auch das nach Destillation rein erhaltene 1,4-Dioxaspiro-[4.15]-eicosan-13-on in die Wittig-Reaktion eingesetzt.

### Beispiel 2:

### 1,4-Dioxaspiro-[4.15]-eicosan-(12/13)-on

Die Synthese wird analog der unter Beispiel 1 angegebenen Vorschrift durchgeführt, nur dass als Edukt 1,8/1,9-Cyclohexadecandion eingesetzt wird. Man erhält ebenfalls ein 3-Komponentengemisch bestehend aus Diketon, Monoacetal und Diacetal, welches als Gemisch oder als reines Monoacetal in die Wittig-Reaktion eingesetzt wird.

¹H-NMR (200 MHz, CDCl₃): δ (ppm) = 1.23-1.39 (m, 16H), 1.52-1.66 (m, 8H), 2.36-2.44 (m, 4H), 3.89-3.94 (m, 4H)
¹³C-NMR (50MHz, CDl₃): δ (ppm) = 22.5, 22.9, 23.0, 23.4, 27.1, 27.2, 27.5, 27.6, 27.7, 27.8, 35.1, 35.2, 41.4, 42,5, 64.3, (2C), 112.1, 212.4.

### Beispiel 3:

### 13-Methylen-1,4-dioxaspiro-[4.15]-eicosan

Man gibt zu einer Suspension aus 29,5 g (250 mmol) Kalium-*tert*-butanolat in 200 ml Diethylether 89,0 g (265 mmol) Methyltriphenylphosphoniumbromid und erhitzt anschließend 15 Minuten unter Rückfluss. Zu der heterogenen Mischung tropft man nun bei 40°C 57,7 g das unter Beispiel 1 hergestellte 3-Komponentengemisch gelöst in 20 ml Diethylether. Jetzt lässt man noch 120 Minuten bei 40°C reagieren, bevor man nach Abkühlen 200 ml Pentan und 300 ml Wasser unter starkem Rühren hinzugibt. Die Phasen werden getrennt und die wässrige Phase wird noch dreimal mit Ether extrahiert. Die vereinigten organischen Phasen werden noch einmal mit Wasser gewaschen, anschließend über Na₂SO₄ getrocknet, abfiltriert und am Rotationsverdampfer vom Lösungsmittel befreit. Nach flashchromatographischer Reinigung (Cyclohexan/EtOAc = 30:1, R_{f} = 0,24) erhält man 22,6 g (84 % über 2 Stufen) eines farblosen Öls.

Die Synthese ausgehend von reinem 1,4-Dioxaspiro-[4.15]-eicosan-13-on wird analog der oben beschriebenen Vorschrift durchgeführt.

### Beispiel 4:

### (12/13)-Methylen-1,4-dioxaspiro-[4.15]-eicosan

Die Synthese erfolgt analog der unter Beispiel 3 beschriebenen Vorschrift, nur das als Edukt ein Isomerengemisch aus 1,4-Dioxaspiro-[4.15]-eicosan-(12/13)-on eingesetzt wird.

¹H-NMR (200 MHz, CDCl₃): δ (ppm) = 1.25-1.48 (m, 20H), 1.54-1.61 (m, 4H) δ (ppm) =1.98-2.08 (m, 4H), 3.90 (s, 2H), 3.91 (s, 2H), 4,70 (s, 2H),
¹³C-NMR (50 MHz, CDCl₃): δ (ppm) = 22.7, 22.8, 26.0, 26.8, 26.9, 27.0, 27.1, 27.2, 27.5, 27.6, 35.0, 35.3, 35.5, 35.6, 64.3 (2C), 109.4, 112.2, 149.7.

### Beispiel 5:

### ((12/13)E/Z)-(12/13)-Ethyliden-1,4-dioxaspiro-[4.15]-eicosan

Die Synthese erfolgt analog der unter Beispiel 3 beschriebenen Vorschrift, nur das als Edukte ein Isomerengemisch aus 1,4-Dioxaspiro-[4.15]-eicosan-(12/13)-on und Ethyltriphenylphosphoniumbromid eingesetzt werden.
Ausbeute: 77 % über zwei Stufen

¹H-NMR (200 MHz, CDCl₃): δ (ppm) = 1.25-1.44 (m, 20H), 1.50-1.64 (m, 7H), 1.90-2.09 (m, 4H), 3.91 (s, 4H), 5.15-5.28 (m, 1H).
¹³C-NMR (50 MHz, CDCl₃): δ (ppm) = 13.2, 22.8, 22.9, 26.3, 26.5, 27.1, 27.3, 27.4, 27.5, 27.6, 27.7, 29.4, 35.5, 35.6, 37.3, 64.3 (2C), 112.2, 118.3, 140.4.

### Beispiel 6:

### 9-Methylencyclohexadecanon

### Variante A:

Man legt 4,1 g (13,9 mmol) 13-Methylen-1,4-dioxaspiro-[4.15]-eicosan in 40 ml Aceton/Wasser = 3:1 (v/v) vor und fügt 780 mg (4,1 mmol) Pyridinium-p-toluolsulfonat hinzu. Jetzt erhitzt man unter Rückfluss bis die Reaktion beendet ist. Nachfolgend rotiert man das Aceton nahezu vollständig ab und gibt 50 ml Diethylether hinzu. Die organische Phase wird je einmal mit ges. NaHCO₃-Lösung und ges. NaCl-Lösung gewaschen, bevor sie über Na₂SO₄ getrocknet und abschließend am Rotationsverdampfer eingeengt wird. Das Rohprodukt wird jetzt mittels Flashchromatographie (Cyclohexan/EtOAc = 25:1, R_{f}= 0,24) gereinigt, so dass man 3,1 g (89 %) eines farblosen Öls bekommt.

### Variante B:

Man gibt zu einer Suspension aus 0,55 g (4,7 mmol) Kalium-*tert*-butanolat und 10 ml Toluol 1,7 g (4,7 mmol) Methyltriphenylphosphoniumbromid. Jetzt erhitzt man 15 Minuten auf 40°C und gibt anschließend 5,0 g (19,8 mmol) 1,9-Cyclohexadecandion, gelöst in 10 ml Toluol, zu. Man läßt nun 120 Minuten bei 40°C reagieren. Nach beendeter Reaktion gibt man 10 ml Pentan und 10 ml Wasser hinzu, trennt die Phasen und extrahiert die wässrige Phase dreimal mit Diethylether. Die vereinigten organischen Phasen werden noch einmal mit Wasser gewaschen, über Na₂SO₄ getrocknet, abfiltriert und am Rotationsverdampfer von Lösungsmittel befreit. Das 3-Komponentengemisch wird mittels Flashchromatographie (Cyclohexan/EtOAc = 25:1, R_{f} = 0,24) getrennt, so dass man 880 mg (75 % bezogen auf eingesetztes Methyltriphenylphosphoniumbromid) 9-Methylencyclohexadecanon erhält.

Geruch: Moschus, erogen, animalisch, ambriert, schöne Muscon-Note, Moschus-Tinktur
¹H-NMR (200 MHz, CDCl₃): δ (ppm) =1.20-1.35 (m, 12H), 1.41 (quint, J = 7.2 Hz, 4H), 1.63 (quint, J = 6.5 Hz, 4H), 2.0 (t, J = 7.2 Hz, 4H), 2.40 (dd, J = 6.5 Hz, 4H), 4.70 (quint, J = 0.9 Hz, 2H).
¹³C-NMR (50 MHz, CDCl₃): δ (ppm) = 23.7 (2C), 26.4 (2C), 27.6 (2C), 27.8 (2C), 27.9 (2C), 35.3 (2C), 42.2 (2C), 109.8, 149.5, 212.5.

### Beispiel 7:

### (8/9)-Methylencyclohexadecanon

Ausgehend von 1,8/1,9-Cyclohexadecandion bzw. 12/13-Methylen-1,4-dioxaspiro-[4.15]-eicosan werden die Synthesen analog der unter Beispiel 6 angegebenen Varianten A und B durchgeführt.

Geruch: Moschus, erogen, animalisch, ambriert, schöne Muscon-Note, Moschus-Tinktur
Angabe des 8-Methylencyclohexadecanons:
¹H-NMR (200 MHz, CDCl₃): δ (ppm) = 1.23-1.35 (m, 12H), 1.36-1.48 (m, 4H), 1.57-1.70 (m, 4H), 2.0 (t, J = 7.5 Hz, 4H), 2.37-2.44 (m, 4H), 4.69 (s, 2H).
¹³C-NMR (50 MHz, CDCl₃): δ (ppm) = 23.4, 23.6, 26.4, 26.5, 27.2, 27.3, 27.5, 27.7 (2C), 27.9, 34.8, 35.3, 41.6 (2C), 109.5, 149.5, 212.4.

### Beispiel 8:

### (8/9)-Ethylidencyclohexadecanon

Die Synthese wird mit ((12/13)E/Z)-(12/13)-Ethyliden-1,4-dioxaspiro-[4.15]-eicosan analog der unter Beispiel 6 angegebenen Variante A durchgeführt.

Geruch: schwach Moschus
Angabe des 9-Ethylidencyclohexadecanons:
¹H-NMR (200 MHz, CDCl₃): δ (ppm) = 1.20-1.44 (m, 16H), 1.55 (d, J = 9.1 Hz, 3H), 1.60-1.75 (m, 4H), 1.85-1.95 (m, 4H), 1.90-2.05 (m, 4H), 5.20 (q, J = 6.8 Hz, 1H).
¹³C-NMR (50 MHz, CDCl₃): δ (ppm) =13.2, 23.8, 23.9, 26.8, 27.1, 27.8, 27.9 (2C), 28.0, 28.1, 28.2, 29.7, 37.4, 42.2, 42.5, 118.9, 140.0, 212.6.

### Beispiel 9:

### 9-Methyl-8(E/Z)-cyclohexadecenon

Man legt 1,3 g (5,1 mmol) 9-Methylencyclohexadecanon in 80 ml Toluol vor und fügt 145 mg (0,75 mmol) p-Toluolsulfonsäure hinzu. Jetzt erhitzt man 44 h auf 100°C, läßt anschließend abkühlen und wäscht die organische Phase einmal mit ges. NaHCO₃-Lösung. Die wässrige Phase wird nun dreimal mit Diethylether extrahiert, bevor die vereinigten organischen Phasen über Na₂SO₄ getrocknet, abfiltriert und von Lösungsmittel befreit werden. Das Rohprodukt wird flashchromatographisch (Cyclohexan/EtOAc = 25:1, R_{f}= 0,23) gereinigt, so dass man 1,25 g (89 %) eines farblosen Öls erhält.

Geruch: Moschus, erogen, animalisch, schöne Muscon-Note, Moschus-Tinktur. Angabe des Überschussisomeren:
¹H-NMR (200 MHz, CDCl₃): δ (ppm) = 1.10-1.48 (m, 14H), 1.53 (s, 3H), 1.57-1.72 (m, 4H), 1.92-2.08 (m, 4H), 2.30-2.46 (m, 4H), 5.11 (t, J = 7.6 Hz, 1H).
¹³C-NMR (50 MHz, CDCl₃): δ (ppm) = 22.9, 23.4,23.9,26.0,26.7,27.1, 27.2, 27.5, 28.0, 28.1, 28.3, 38.5, 40.0, 42.5, 125.7, 134.5, 212.7.

### Beispiel 10:

### 8-Methyl-(E/Z)-7/-(E/Z)-8-cyclohexadecenon/9-Methyl-(E/Z)-8-cyclohexadecenon

Die Synthese verläuft analog der unter Beispiel 9 beschriebenen Vorschrift, mit der Änderung, dass als Edukt (8/9)-Methylencyclohexadecanon eingesetzt wird.

Geruch: Moschus, erogen, animalisch, schöne Muscon-Note, Moschus-Tinktur. Angabe des Überschussisomeren:
¹H-NMR (200 MHz, CDCl₃): δ (ppm) = 1.12-1.46 (m, 12H), 1.54 (s, 3H), 1.58-1.71 (m, 4H), 1.90-2.08 (m, 4H), 2.31-2.46 (m, 4H), 5.0-5.17 (m, 1H).
¹³C-NMR (50 MHz, CDCl₃): δ (ppm) = 15.3, 22.9, 23.9, 26.7, 27.1, 27.2, 27.5, 28.0, 28.1 (2C), 28.3, 38.5, 40.0, 42.5, 125.8, 134.5, 212.7.

### Beispiel 11:

### 8-Ethyl-(E/Z)-7/-(E/Z)-8-cyclohexadecenon/9-Ethyl-(E/Z)-8-cyclohexadecenon

Die Synthese verläuft analog der unter Beispiel 9 beschriebenen Vorschrift, mit der Änderung, dass als Edukt (8/9)-Ethylidencyclohexadecanon eingesetzt wird.

Geruch: schwach Moschus
Angabe des Überschussisomeren:
¹H-NMR (200 MHz, CDCl₃): δ (ppm) = 0.96 (t, J = 8.1 Hz, 3H), 1.14-1.44 (m, 14H), 1.54-1.72 (m, 4H), 1.91-2.10 (m, 6H), 2.22-2.47 (m, 4H), 4.95-5.27 (m, 1H).
¹³C-NMR (50 MHz, CDCl₃): δ (ppm) = 12.9, 23.1, 24.1, 27.4, 27.5, 27.7, 27.8, 28.0, 28.1, 28.3, 29.4, 29.6, 37.4, 40.9, 43.1, 123.6, 141.0, 212.8.

### Beispiel 12:

### 9-Methylcyclohexadecanon

Man legt 500 mg (2 mmol) 9-Methylencyclohexadecanon in 25 ml Essigsäureethylester vor und fügt 25 mg Pd/C hinzu. Jetzt wird bei 40°C und Normalwasserstoffdruck 5 h hydriert. Nach beendeter Reaktion filtriert man über Celite ab und befreit das Rohprodukt von Lösungsmittel. Nach flashchromatographischer Reinigung (Cyclohexan/EtOAc = 25:1) erhält man 475 mg (94%) eines farblosen Öls.

Geruch: Moschus, erogen, animalisch, schwach.
¹H-NMR (200 MHz, CDCl₃): δ (ppm) = 0.84 (d, J = 7.2 Hz, 3H), 1.06-1.18 (m, 2H), 1.20-1.36 (m, 18H), 1.39-1.49 (m, 1H), 1.55-1.64 (m, 2H), 1.64-1.74 (m, 2H), 2.34 (ddd, J =16.0, 7.8, 6.0 Hz, 2H), 2.48 (ddd, J =16.0, 7.6, 6.0 Hz, 2H).
¹³C-NMR (50 MHz, CDCl₃): δ (ppm) = 21.0, 23.5 (2C), 24.6 (2C), 27.5 (2C), 27.6 (2C), 27.7 (2C), 30.6,34.4 (2C), 42.1 (2C), 212.5.

### Beispiel 13:

### (8/9)-Methylcyclohexadecanon

Die Synthese verläuft analog der unter Beispiel 12 beschriebenen Vorschrift, mit der Änderung, dass als Edukt (8/9)-Methylencyclohexadecanon eingesetzt wird.

Geruch: Moschus, erogen, schwach.
Angabe des 8-Methylcyclohexadecanon-Isomeren
¹H-NMR (200 MHz, CDCl₃): δ (ppm) = 0.84 (d, J = 7.2 Hz, 3H), 1.08-1.18 (m, 2H), 1.18-1.36 (m, 18H), 1.40-1.50 (m, 1H), 1.55-1.74 (m, 4H), 2.30-2.50 (m, 4H).
¹³C-NMR (50 MHz, CDCl₃): δ (ppm) = 20.7, 23.3, 23.6, 24.6, 24.8, 27.2, 27.3, 27.4 (2C), 27.7, 27.8, 30.4, 34.0 (2C), 41.9, 42.0, 212.4.

### Beispiel 14:

### (8/9)-Ethylcyclohexadecanon

Die Synthese verläuft analog der unter Beispiel 12 beschriebenen Vorschrift, mit der Änderung, dass als Edukt (8/9)-Ethylencyclohexadecanon eingesetzt wird.

Geruch: schwach Moschus
Angabe des 9-Ethylcyclohexadecanon-Isomeren:
¹H-NMR (200 MHz, CDCl₃): δ (ppm) = 0.84 (t, J = 8.0 Hz, 3H), 1.14-1.38 (m, 23H), 1.52-1.74 (m, 4H), 2.30-2.51 (m, 4H).
¹³C-NMR (50 MHz, CDCl₃): δ (ppm) = 11.6, 23.2, 23.6 (2C), 24.6 (2C), 27.6 (2C), 27.8 (2C), 27.9 (2C), 31.7 (2C), 37.9,42.3 (2C), 212.6.

### Beispiel 15:

Das vorliegende Parfümöl dient zur Parfümierung vielerlei kosmetischer Produkte.

### Zusammensetzung:

| **Ingredienzien** | **Gewichtsteile** |
|---|---|
| 1. Citrophoral Base (H&R) | 5,0 |
| 2. Aldehyd C10 10 % in BA | 5,0 |
| 3. Aldehyd C11 MOA 10 % in BA | 3,0 |
| 4. Farenal (H&R) | 3,0 |
| 5. Aldehyd C11 10 % in IPM | 5,0 |
| 6. Citroxal 50 % in DEP | 2,0 |
| 7. trans Hex-2-enol 10 % in BA | 2,0 |
| 8. Vertocitral (H&R) | 1,0 |
| 9. Linalylacetat | 45,0 |
| 10. Citrylal (H&R) | 5,0 |
| 11. Mandarinal (Firmenich) | 4,0 |
| 12. Lilial (Givaudan Roure) | 75,0 |
| 13. Lyral (IFF) | 75,0 |
| 14. Profamesol (H&R) | 5,0 |
| 15. Nerolidol | 5,0 |
| 16. Linalool | 45,0 |
| 17. Geraniumöl afrikanisch | 5,0 |
| 18. Phenylethylalkohol | 75,0 |
| 19. Geraniol | 15,0 |
| 20. Nerol | 10,0 |
| 21. Hexylzimtaldehyd alpha | 50,0 |
| 22. Methyldihydrojasmonat | 15,0 |
| 23. Benzylsalicylat | 100,0 |
| 24. trans,cis-2-Nonadienol 0,1 % in IPM | 5,0 |
| 25. Allylionon (Givaudan Roure) | 3,0 |
| 26. Isomethylionon gamma | 75,0 |
| 27. Eugenol | 7,0 |
| 28. Cedrylacetat | 40,0 |
| 29. Sandolen (H&R) | 5,0 |
| 30. Citral | 5,0 |
| BA = Benzylalkohol; IPM = Isopropylmyristat; DEP = Diethylphthalat | |

Der Zusatz von
a) 355 Gewichtsteilen 8/9-Methylencyclohexadecanon (Summe 1000 Gewichtsteile) führt zu einer deutlich wahrnehmbaren Harmonisierung der frischen Kopfnote mit der rosig-blumigen Herznote. Darüber hinaus werden mit 8/9-Methylencyclohexadecanon an Nitromoschus erinnernde Effekte erzielt und die feine erogene Moschusnote verleiht der vorliegenden Komposition eine hervorragende Ausstrahlung und gesteigerte Haftung. Hierbei setzt sich besonders der wertvolle Charakter von 8/9-Methylencyclohexadecanon im Vergleich zu Kompositionen mit konventionellen Moschusriechstoffen durch.
b) 55 Gewichtsteilen 9-Methyl-8(E/Z)-cyclohexadecenon (Summe 700 Gewichtsteile) verleiht der Komposition eine animalische Moschusnote, die mit existierenden Moschusriechstoffen nicht erreicht wird. Weiterhin gewinnt die gesamte Komposition an Fülle und erscheint wertvoller.

## Patentansprüche

1. Makrocyclischen Ketone der allgemeinen Formel 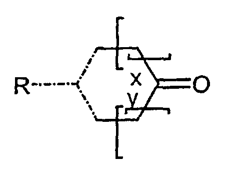 worin
R eine C₁-C₆-Alkyl- oder C₁-C₆-Alkylidengruppe bedeutet,
x = 5 und y = 7, oder x = 6 und y = 6 sind, und
die unterbrochenen Linien unabhängig voneinander C-C-Einfach- oder eine C=C-Doppelbindung
bedeuten.

2. Makrocyclischen Ketone nach Anspruch 1 der Formel 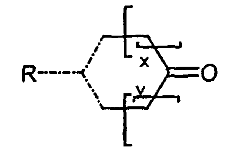 worin
R Methyl oder Ethyl bedeutet und
x und y und die unterbrochenen Linien die oben genannte Bedeutung haben.

3. 8-Methylencyclohexadecanon, 9-Methylencyclohexadecanon, 8-Ethylencyclohexadecanon, 9-Ethylencyclohexadecanon, 8-Methyl-(E/Z)-7/-(E/Z)-8-cyclohexadecenon, 9-Methyl-(E/Z)-8-cyclohexadecenon, 8-Ethyl-(E/Z)-7/-(E/Z)-8-cyclohexadecenon, 9-Ethyl-(E/Z)-8-cyclohexadecenon, 8-Methylcyclohexadecanon, 9-Methylcyclohexadecanon, 8-Ethylcyclohexadecanon und 9-Ethylcyclohexadecanon.

4. Riechstoffkompositionen, enthaltend makrocyclische Ketone nach Anspruch 1.

5. Verwendung von makrocyclischen Ketonen Nach Anspruch 1 als Riechstoff mit Muscon-Note.

6. Verfahren zur Herstellung der Makrocyclischen Ketone nach Anspruch 1, der Formel 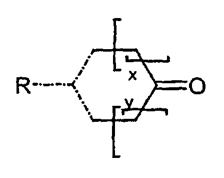 worin
die unterbrochenen Linien unabhängig voneinander C-C-Einfach- oder eine C=C-Doppelbindung bedeuten
R eine C₁-C₆-Alkyl- oder C₁-C₆-Alkylidengruppe bedeutet,
x = 5 und y = 7, oder
x = 6 und y = 6 sind,
das **dadurch gekennzeichnet ist, dass** als Edukt Cyclohexadecandion der Formel 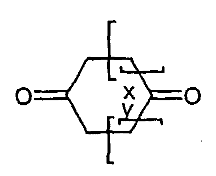 worin
x und y die oben genannte Bedeutung haben,
eingesetzt wird,
und in einem ersten Schritt mit einem C₁-C₆-Alkyl-Triphenylphosphoniumhalogenid und einer starken Base in einem aprotischen Lösungsmittel umgesetzt wird und die so erhaltenen C₁-C₆-Alkylidencyclohexadecanone gegebenenfalls isomerisiert und hydriert werden.

7. Verfahren zur Herstellung der Makrocyclischen Ketone nach Anspruch 6 das **dadurch gekennzeichnet ist, dass** in einem ersten Schritt eine Ketofunktion über ein Ethylenacetal geschützt wird, nachfolgend eine Wittig-Reaktion durchgeführt wird und die Schutzgruppe abgespalten wird und in weiteren Schritten gegebenenfalls eine Isomerisierung und Hydrierung erfolgt.

## Claims

1. Macrocyclic ketones of the general formula 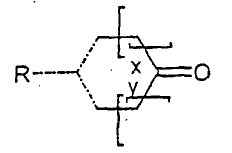 where
R denotes a C₁-C₆-alkyl or C₁-C₆-alkylidene group,
x = 5 and y = 7, or
x = 6 and y = 6, and
the broken lines independently of one another denote [lacuna] C-C single bond or a C=C double bond.

2. Macrocyclic ketones according to Claim 1, of the formula 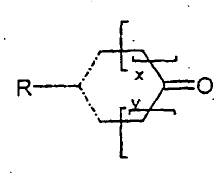 where
R denotes methyl or ethyl and
x and y and the broken lines have the abovementioned meaning.

3. 8-methylenecyclohexadecanone, 9-methylenecyclohexadecanone, 8-ethylenecyclohexadecanone, 9-ethylenecyclohexadecanone, 8-methyl-(E/Z)-7/(E/Z)-8-cyclohexadecenone, 9-methyl-(E/Z)- 8-cyclohexadecenone, 8-ethyl-(E/Z)-7/-(E/Z)-8-cyclohexadecenone, 9-ethyl-(E/Z)- 8-cyclohexadecenone, 8-methylcyclohexadecanone, 9-methylcyclohexadecanone, 8-ethylcyclohexadecanone and 9-ethylcyclohexadecanone

4. Fragrance compositions containing macrocyclic ketones according to Claim 1.

5. Use of macrocyclic ketones according to Claim 1 as a fragrance with muscone note.

6. Method for the preparation of the macrocyclic ketones according to Claim 1 of the formula 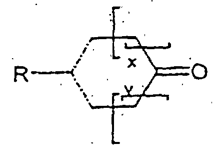 where
the broken lines independently of one another denote [lacuna] C-C single bond or a C=C double bond.
R denotes a C₁-C₆-alkyl or C₁-C₆-alkylidene group,
x = 5 and y = 7, or
x = 6 and y = 6,
that is **characterised in that** cyclohexadecanedione of the formula 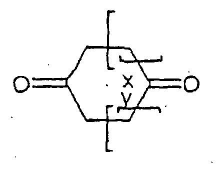 where
x and y have the abovementioned meaning,
is used as educt,
and in a first step is reacted with a C₁-C₆-alkyl-triphenylphosphonium halide and a strong base in an aprotic solvent and the C₁-C₆-alkylidenecyclohexadecanones thus obtained are optionally isomerised and hydrogenated.

7. Method for the preparation of the macrocyclic ketones according to Claim 6 that is **characterised in that** in a first step a ketone group is protected via an ethylene acetal, a Wittig reaction is then carried out and the protective group is split off and an isomerisation and hydrogenation optionally take place in further steps.

## Revendications

1. Cétones macrocycliques répondant à la formule générale : 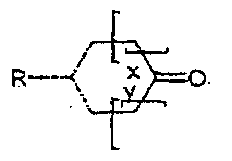 dans laquelle
R représente un groupe alkyle en C₁-C₆ ou un groupe alkylidène en C₁-C₆,
X = 5 et y = 7, ou
x = 6 et y = 6, et
les lignes discontinues représentent, indépendamment les unes des autres, une liaison simple C-C ou une double liaison C=C.

2. Cétones macrocycliques selon la revendication 1, répondant à la formule 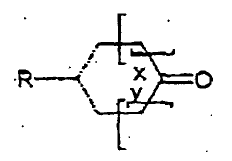 dans laquelle
R représente un groupe méthyle ou un groupe éthyle et
x et y et les lignes discontinues ont la signification mentionnée précédemment.

3. 8-méthylènecyclohexadécanone, 9-méthylènecyclohexadécanone, 8-éthylènecyclohexadécanone, 9-éthylènecyclohexadécanoné, 8-méthyl-(E/Z)-7/-(E/Z)-8-cyclohexadécénone, 9-méthyl-(E/Z)-8-cyclohexadécénone, 8-éthyl-(E/Z)-7/-(E/Z)-8-cyclohexadécénone, 9-éthyl-(E/Z)-8-cyclohexadécénone, 8-méthylcyclohexadécanone, 9-méthylcyclohexadécanone, 8-éthylcyclohexadécanone et 9-éthylcyclohexadécanone

4. Compositions odoriférantes, contenant des cétones macrocycliques selon la revendication 1.

5. Utilisation de cétones macrocycliques selon la revendication 1, en tant que substance odoriférante avec une note de muscone.

6. Procédé pour la fabrication des cétones macrocycliques selon la revendication 1, répondant à la formule, 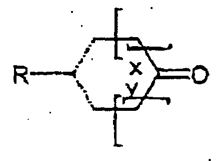 dans laquelle
les lignes discontinues représentent, indépendamment les unes des autres, une liaison simple C-C ou une double liaison C=C,
R représente un groupe alkyle en C₁-C₆ ou un groupe alkylidène en C₁-C₆,
x = 5 et y = 7, ou
x = 6 et y = 6, et
**caractérisé en ce que** l'on utilise comme produit d'addition la cyclohexadécandione répondant à la formule, 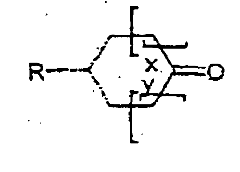 dans laquelle x et y ont la signification mentionnée précédemment,
et **en ce que**, au cours d'une première étape, on fait réagir ledit produit d'addition avec un halogénure de triphénylphosphonium d'alkyle en C₁-C₆ et une forte base dans un solvant aprotique et **en ce que** la cyclohexadécanone d'alkylidène en C₁-C₆ ainsi obtenue est isomérisée et hydrogénée, le cas échéant.

7. Procédé pour la fabrication des cétones macrocycliques selon la revendication 6, **caractérisé en ce que**, dans une première étape, une fonction céto est protégée par un acétal d'éthylène, une réaction du type Wittig se produit ensuite et le groupe de protection se sépare et dans des étapes ultérieures une isomérisation et hydrogénation ont lieu, le cas échéant.
